# FASCICULE DE BREVET EUROPEEN

(11) **EP 4 014 934 B1**
(45) Date de publication et mention de la délivrance du brevet: **08.03.2023**
(21) Numéro de dépôt: 21212308.7
(22) Date de dépôt: 03.12.2021
(51) Int. Cl.: A61B 17/00, A61F 7/12, A61F 7/00, A61N 5/10

(54) **DISPOSITIF DE REFROIDISSEMENT LOCALISÉ**
VORRICHTUNG ZUR LOKALISIERTEN KÜHLUNG
LOCALISED COOLING DEVICE

(30) Priorité: 10.12.2020 FR 2012954
(43) Date de publication de la demande: 22.06.2022
(73) Titulaire: Commissariat à l'énergie atomique et aux énergies alternatives, 75015 Paris (FR)
(72) Inventeur: CHABROL, Claude, 38054 Grenoble cedex 09 (FR); BORNTRAGER, Quentin, 38054 Grenoble cedex 09 (FR); FULBERT, Clémentine, 38054 Grenoble cedex 09 (FR); RATEL, David, 38054 Grenoble cedex 09 (FR)
(74) Mandataire: INNOV-GROUP

(56) Documents cités:
- EP-A2- 0 970 724
- US-A1- 2002 087 206
- US-A1- 2002 123 783
- US-A1- 2004 077 931
- US-A1- 2006 111 765
- US-A1- 2020 222 676
- US-B2- 6 849 072

## Description

### Domaine technique de l'invention

La présente invention se situe dans le domaine des dispositifs de refroidissement localisé qui comportent au moins un élément expansible destiné à venir se loger dans une cavité d'exérèse, suite à l'ablation chirurgicale d'une tumeur réalisée chez un être vivant, pour refroidir tout ou partie des parois de cette cavité.

### Etat de la technique

Actuellement, les glioblastomes sont les tumeurs intracrâniennes les plus fréquentes chez l'adulte, avec une incidence de plus de cinq nouveaux cas par an en France pour 100 000 habitants. Le protocole de traitement standard actuellement utilisé a été défini par l'équipe de Stupp et se compose d'une ablation chirurgicale suivie de radiothérapie et de chimiothérapie administrées en concomitance.

Malgré l'ensemble de ces traitements, la tumeur récidive chez plus de 90% des patients. Cette récidive est principalement locale et a lieu dans les premiers millimètres entourant la cavité d'exérèse, en raison du caractère infiltrant des glioblastomes. A ce jour, bien que certaines thérapies permettent de retarder la progression tumorale, aucun traitement n'a montré de réelle efficacité sur la récidive de ce type de tumeurs.

Des solutions antérieures proposent des dispositifs qui permettent un refroidissement global du cerveau, notamment du liquide cérébrospinal comme dans les brevets US7004961B2 et US7156867B2, ne permettant donc pas un traitement localisé.

D'autres solutions ont déjà été explorées pour traiter les tissus entourant une cavité d'exérèse, comme dans les demandes de brevet WO2019/147210A1 et WO9222350A1. Ces solutions ont pour objectif la diffusion localisée de principes actifs dans les tissus qui entourent la cavité et permettent éventuellement de réaliser un apport de chaleur. Cependant, il n'a pas été démontré qu'elles permettaient de diminuer les risques de récidive.

D'autres solutions ont également été décrites dans les demandes de brevet US2002/087206A1, EP0970724A2 et US2020/222676A1.

Le but de l'invention est de proposer un dispositif permettant de réaliser un refroidissement localisé d'une cavité d'exérèse après l'ablation chirurgicale d'une tumeur, tout en minimisant les risques médicaux lors de l'implantation. Ce dispositif vise à être implanté dans la cavité d'exérèse immédiatement après la chirurgie. Le refroidissement est démarré le plus tôt possible, afin de réduire le risque inflammatoire lié à la chirurgie et d'empêcher une reprise de la croissance tumorale.

L'objectif de l'invention proposée est de retarder voire d'empêcher la récidive locale d'une tumeur, de limiter l'inflammation ou encore de proposer un traitement anticancéreux local.

Les données expérimentales acquises récemment ont permis de confirmer l'hypothèse que l'hypothermie modérée pouvait inhiber le développement de cellules tumorales, notamment en bloquant leur prolifération et en réduisant leur migration.

L'invention cible les tumeurs solides avec un fort potentiel de récidive locale, notamment les tumeurs cérébrales telles que le glioblastome. Cependant, elle peut également s'appliquer à d'autres tumeurs solides comme certains carcinomes mammaires, certains carcinomes du colon, ou encore certains cancers de la vessie, de l'ovaire et de la thyroïde.

Le but de l'invention est donc de proposer un dispositif permettant un refroidissement localisé efficace d'une cavité d'exérèse. Ce dispositif doit pouvoir être implanté durablement et éventuellement permettre également l'injection d'agents thérapeutiques. Il permettra également d'obtenir une température la plus uniforme possible au niveau des parois de la cavité d'exérèse.

### Exposé de l'invention

Ce but est atteint par un dispositif de refroidissement localisé comprenant un élément expansible destiné à occuper le volume d'une cavité d'exérèse suite à l'ablation chirurgicale d'une tumeur réalisée chez un être vivant, ledit élément expansible présentant une paroi externe destinée à venir en contact au moins partiellement avec les parois de la cavité, le dispositif comportant :
- Une unité de refroidissement comprenant un circuit fluidique dans lequel peut circuler un fluide de refroidissement, un module de refroidissement coopérant avec ledit circuit fluidique et au moins un ensemble de pompage connecté sur ledit circuit fluidique pour faire circuler le fluide dans ledit circuit fluidique,
- Une unité de commande et d'alimentation électrique pour commander et alimenter ladite unité de refroidissement,
- Ledit élément expansible comportant une cavité interne délimitée par une paroi,
- Un conduit en serpentin, appartenant au circuit fluidique et rapporté sur ladite paroi, formant ladite paroi de l'élément expansible, ou logé dans la cavité interne de l'élément expansible.

Selon une particularité, ladite paroi de la cavité présente une structure alvéolée réalisée par le conduit en serpentin.

Selon une autre particularité, le conduit en serpentin est réalisé sous la forme de deux spirales imbriquées.

Selon une autre particularité, le conduit en serpentin est réalisé sous la forme d'un tube bi-lumen.

Selon une autre particularité, l'élément expansible comporte un ballonnet interne présentant une surface externe sur laquelle est rapporté ledit conduit en serpentin et un ballonnet externe venant recouvrir ledit conduit en serpentin.

Selon une autre particularité, le conduit en serpentin comporte une entrée et une sortie et sa sortie débouche dans ladite cavité interne de l'élément expansible.

Selon une variante de réalisation, ledit conduit en serpentin est logé dans ladite cavité interne de l'élément expansible.

Selon une autre particularité, le dispositif comporte également un circuit indépendant de diffusion d'un fluide thérapeutique connecté sur ledit ensemble de pompage et agencé sur ou autour de la paroi externe dudit élément expansible.

Selon une réalisation particulière, ledit circuit de diffusion de fluide thérapeutique comporte un conduit rapporté sur la paroi externe dudit élément expansible.

Selon une autre réalisation particulière, ledit circuit de diffusion de fluide thérapeutique comporte un ballonnet externe solidaire dudit élément expansible.

Selon une autre particularité, l'élément expansible est thermoformé pour présenter une forme externe de dimensions identiques à celles de la cavité d'exérèse.

Selon une autre particularité, le module de refroidissement comporte un module à effet Peltier.

Selon une autre particularité, le dispositif comporte des moyens de suivi de la température du fluide et du module de refroidissement, de débit et de pression de fluide de refroidissement dans le circuit fluidique, et de volume et de la turbidité du fluide de refroidissement.

Selon une autre particularité, l'ensemble de pompage est actionné de manière fluidique.

Selon une autre particularité, le dispositif comporte un boîtier hermétique logeant le module de refroidissement et l'ensemble de pompage.

Selon une autre particularité, l'élément expansible est fixé audit boîtier, un câble reliant ledit boîtier à l'unité de commande et d'alimentation, via un connecteur transcutané.

Selon une autre particularité, le dispositif comporte un réservoir destiné à recevoir un fluide et relié au circuit fluidique, et un actionneur pilotable par l'unité de commande et d'alimentation pour régler le niveau de fluide dans le réservoir.

Selon une autre particularité, le dispositif comporte des moyens d'isolation thermique positionnés contre au moins une partie de la paroi externe de l'élément expansible.

### Brève description des figures

D'autres caractéristiques et avantages vont apparaître dans la description détaillée qui suit faite en regard des dessins annexés dans lesquels :
- La figure 1A et la figure 1B représentent de manière schématique deux architectures du dispositif de refroidissement conforme à l'invention ;
- La figure 2A et la figure 2B représentent un exemple de réalisation de l'élément expansible employé dans le dispositif de refroidissement de l'invention ;
- La figure 3A et la figure 3B représentent une variante de réalisation de l'élément expansible employé dans le dispositif de refroidissement de l'invention ;
- La figure 4A, la figure 4B, la figure 4C, la figure 4D et la figure 4E montrent des vues en coupe de plusieurs variantes de réalisation de la paroi de l'élément expansible ;
- La figure 5 illustre une méthode de thermoformage de l'élément expansible employé dans le dispositif de refroidissement de l'invention ;
- La figure 6 représente un mode de réalisation particulier du module de refroidissement employé dans le dispositif de refroidissement de l'invention ;
- La figure 7 représente un exemple de réalisation du dissipateur thermique employé dans le dispositif de refroidissement de l'invention ;
- La figure 8 montre une variante de réalisation du dispositif de refroidissement de l'invention permettant l'administration d'un fluide thérapeutique ;
- La figure 9A, la figure 9B et la figure 9C représentent trois modes de réalisation du dispositif de refroidissement de l'invention ;
- La figure 10A et la figure 10B illustrent deux variantes d'implantation du dispositif de refroidissement de l'invention ;
- La figure 11 représente un deuxième mode de réalisation préféré du dispositif de refroidissement de l'invention ;
- La figure 12 montre une variante de réalisation du dispositif de refroidissement de l'invention ;
- La figure 13A, la figure 13B et la figure 13C illustrent trois variantes de réalisation d'une solution d'isolation thermique de l'élément expansible par rapport à certaines parois de la cavité d'exérèse ;
- La figure 14 représente une variante de réalisation de l'élément expansible employé dans l'invention ;
- La figure 15 montre un diagramme illustrant l'influence de différentes températures sur la prolifération de cellules de glioblastome ;
- La figure 16 montre un diagramme illustrant l'influence d'une hypothermie de 28°C sur la prolifération de cellules de glioblastome au cours du temps ;
- La figure 17 montre un diagramme illustrant l'influence d'une hypothermie de 28°C sur la viabilité des cellules de glioblastome au cours du temps ;
- La figure 18 montre un diagramme illustrant l'influence d'une hypothermie de 28°C sur la migration de cellules de glioblastome ;

### Description détaillée d'au moins un mode de réalisation

L'invention vise à proposer un dispositif 1 implantable durablement en profondeur et dédié au refroidissement localisé d'une cavité d'exérèse (référencée CE sur les figures annexées), par exemple réalisée dans le cerveau (référencé C) d'un être vivant. Le refroidissement des tissus peut avoir différents objectifs :
- Limiter localement l'inflammation due au contexte thérapeutique invasif,
- Inhiber la progression tumorale et notamment la récidive, et
- Potentialiser les effets d'autres traitements tels que la chimiothérapie ou la radiothérapie.

Ce dispositif 1 doit minimiser les risques médicaux liés à son utilisation, notamment le risque infectieux, tout en étant compatible avec le quotidien d'un patient pour une implantation long terme (par exemple au moins trente jours).

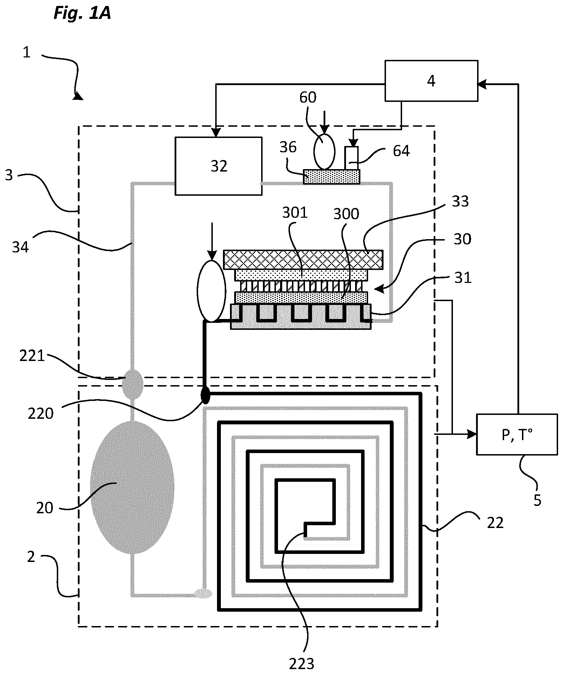

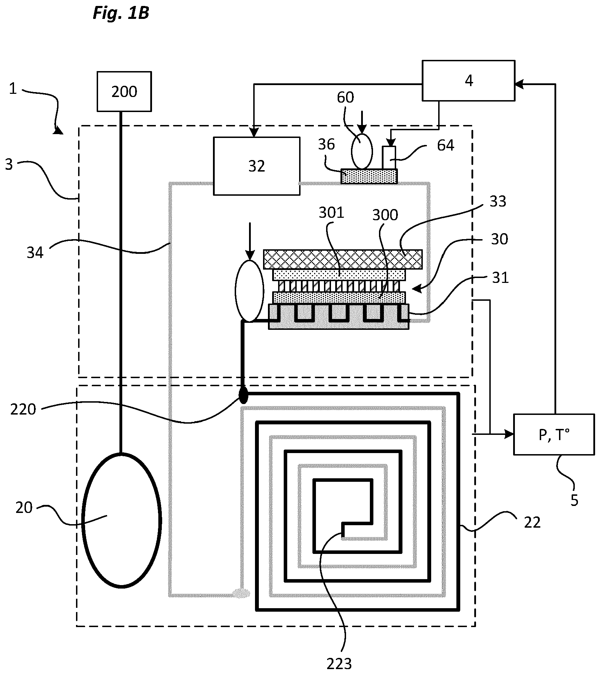

En référence à la figure 1A et à la figure 1B, le dispositif de l'invention comporte principalement :
- Un élément expansible 2 destiné à venir se loger dans la cavité d'exérèse CE,
- Une unité de refroidissement 3,
- Une unité de commande et d'alimentation électrique 4.

On verra que différents moyens de surveillance 5 peuvent également être prévus dans le dispositif 1 pour optimiser son fonctionnement.

L'élément expansible 2 est destiné à venir se loger dans la cavité d'exérèse CE préalablement formée. Il peut être rétractable pour pouvoir être inséré dans la cavité d'exérèse puis gonflé une fois en place. Il est destiné à venir en contact avec l'ensemble des parois de la cavité d'exérèse.

L'élément expansible 2 peut être fabriqué en silicone de faible dureté et de faible épaisseur, afin d'en faire un objet conformable à la cavité en appliquant le minimum de pression. Pour son utilisation en clinique, plusieurs tailles pourront être proposées en fonction du volume de la cavité d'exérèse, afin d'éviter d'avoir à appliquer une pression trop élevée, et d'éviter les risques en cas de défaillance.

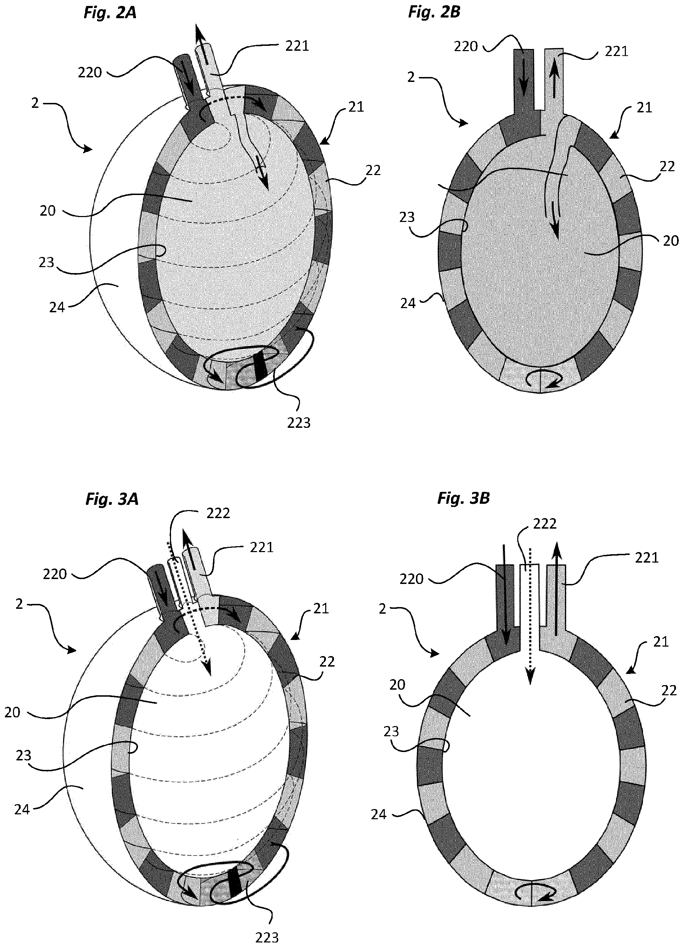

En référence à la figure 2A et à la figure 2B, l'élément expansible 2 comporte une paroi 21 présentant une structure alvéolée, délimitant une cavité interne 20. Cette structure alvéolée est utilisée pour garantir un refroidissement homogène des parois de la cavité d'exérèse CE.

La paroi 21 est réalisée à l'aide d'un conduit 22 agencé en serpentin. Le conduit 22 en serpentin peut suivre un profil suivant deux spirales imbriquées, formant la structure alvéolée de la paroi 21. Ce conduit 22 en serpentin fait partie d'un circuit fluidique. Il comporte une entrée 220 dans laquelle est injecté un fluide de refroidissement ou un fluide thérapeutique et une sortie 221.

De manière non limitative, le conduit 22 en serpentin, partant de son entrée 220, comporte une première portion formant un circuit aller en spirale et une deuxième portion formant un circuit retour en spirale, imbriqué dans le circuit aller et menant jusqu'à la sortie 221. L'entrée et la sortie sont par exemple situées du même côté de l'élément expansible 2, de manière juxtaposée. Les deux circuits sont connectés entre eux par une extrémité 223 commune.

Comme illustré par la figure 1A, la figure 2A et la figure 2B, la cavité interne 20 de l'élément expansible peut être connectée en série sur la sortie 221 du conduit en serpentin. L'élément expansible 2 peut ainsi être gonflé par le fluide injecté dans le circuit. Sur la figure 1A, le conduit 22 en serpentin est représenté à plat et la cavité interne 20 est montrée connectée en série avec le conduit 22. En variante illustrée par la figure 1B, la figure 3A et la figure 3B, le conduit et la cavité peuvent être réalisés suivant deux circuits distincts, l'élément expansible 2 étant alors gonflé de manière indépendante par injection d'un fluide (eau ou air par exemple, via une pompe 200) dans sa cavité interne 20, via une entrée 222 débouchant directement dans cette cavité interne 20 de l'élément expansible 2. De manière non limitative, la pompe 200 peut faire partie de l'unité de commande et d'alimentation électrique 4.

Sur la figure 2A, la figure 2B, la figure 3A et la figure 3B, le circuit dit "froid" est représenté en gris foncé et le circuit de retour dit "chaud" est représenté en gris clair. Les flèches noires illustrent le sens de circulation du liquide de refroidissement dans l'élément. Sur la figure 3A et la figure 3B, la flèche en pointillés indique l'injection d'un fluide tel que l'eau ou l'air dans la cavité interne 20 de l'élément, via l'entrée spécifique 222.

Selon un aspect particulier de l'invention, lorsque le fluide de refroidissement est injecté dans le conduit 22 en serpentin, celui-ci se diffuse ainsi de manière homogène dans toute la structure alvéolée de la paroi 21 de l'élément expansible 2, en continu dans le circuit aller puis dans le circuit retour, permettant d'obtenir un refroidissement uniforme de la cavité d'exérèse.

L'élément expansible présente avantageusement une forme adaptée à celle de la cavité d'exérèse CE dans laquelle il vient se loger. Sur la figure 2A, la figure 2B, la figure 3A et la figure 3B, il est représenté avec une forme sphérique mais il faut comprendre que, selon la forme de la cavité, il va venir au mieux épouser la forme de cette cavité. Le conduit en serpentin est alors enroulé autour de l'axe de révolution de l'élément expansible 2.

De manière non limitative, l'élément expansible 2 peut être formé par l'assemblage d'un ballonnet interne 23 délimitant la cavité interne 20, le conduit 22 en serpentin étant rapporté sur la surface externe dudit ballonnet interne et un ballonnet externe 24 venant recouvrir le conduit 22 en serpentin.

Le ballonnet 24 externe peut être réalisé, par exemple, en polyuréthane (PU) afin d'être thermoformé pour s'adapter au mieux à la cavité d'exérèse CE. Certains matériaux PU sont compatibles avec une stérilisation gamma (40 à 50 kGy), ce qui assure une compatibilité du ballonnet avec la radiothérapie, dont l'irradiation est de 60 Gy.

Une alternative à la réalisation du ballonnet externe 24 en polyuréthane est la réalisation d'un composite silicone (extensible) armé par des fils biocompatibles et thermoformables (polypropylène, polyamide ou polyester par exemple). La température de mise en oeuvre (respectivement 165°C, 205°C et 225°C) est équivalente à celle du polyuréthane, et compatible avec un enrobage silicone (température d'utilisation »250°C). Du fil de plastique thermodurcissable fin (100µm à 200µm par exemple) peut être déposé avec un maillage adapté à l'utilisation (souplesse suffisante en permettant l'expansion sans formation d'hernie) simplement par impression 3D selon la forme souhaitée, ce maillage étant surmoulé ultérieurement par un film de silicone pour former le ballonnet. Ce ballonnet pourra être thermoformé en respectant certaines limites de déformation maximum, et ainsi limiter l'expansion à une forme correspondant au volume d'exérèse tout en restant fin (pour limiter les pertes thermiques) et souple.

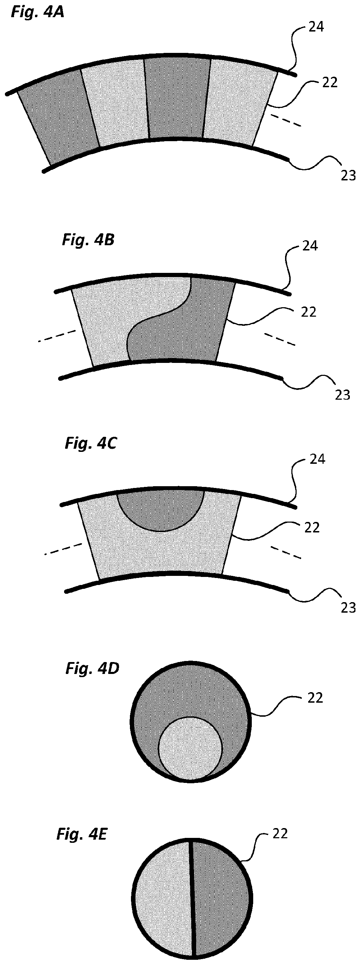

La figure 4A, la figure 4B et la figure 4C montrent, grâce à plusieurs vues en coupe, différentes variantes de réalisation de la paroi 21 à structure alvéolée de l'élément expansible 2. Comme représenté sur la figure 4A, le circuit aller et le circuit retour peuvent être réalisés par l'assemblage d'un ou plusieurs tubes fixés entre eux. Les tubes peuvent être de type bi-lumen, afin de faciliter l'assemblage du ballonnet alvéolé. La figure 4B, la figure 4C, la figure 4D et la figure 4E montrent plusieurs variantes non limitatives de réalisation du conduit 22 à partir d'un seul tube bi-lumen, intégrant le circuit aller et le circuit retour. Bien entendu, d'autres variantes pourraient être envisagées.

La réalisation et l"assemblage des différents composants de l'élément expansible peuvent être réalisés par extrusion, enduction par trempage ("Dip coating" en anglais) et/ou collage.

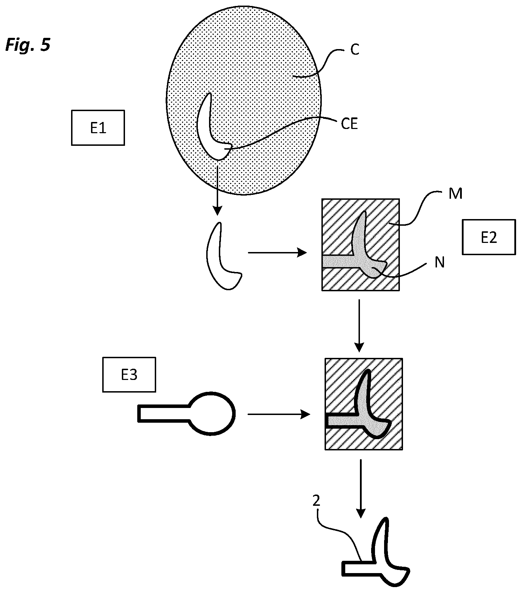

Selon une réalisation particulière, l'élément expansible peut être réalisé par thermoformage, après un moulage de la cavité d'exérèse CE, par exemple réalisé en impression 3D. Ce principe est illustré par la figure 5. Dans une première étape E1, une image IRM de la cavité d'exérèse CE est réalisée. Cette image est segmentée afin de fournir un fichier 3D de la cavité d'exérèse CE. Un moule M de la CE est réalisé par impression 3D (étape E2). Dans une troisième étape E3, le matériau de fabrication de l'élément expansible 2 est mis en place dans le moule puis thermoformé en le gonflant à l'aide d'air chaud : II prend alors la forme de la cavité d'exérèse CE.

L'unité de refroidissement 3 du dispositif comporte un module de refroidissement 30 et un circuit fluidique 34, dans lequel est amené à circuler un fluide de refroidissement ou un fluide thérapeutique, et un ensemble de pompage 32 commandé pour assurer la circulation du fluide dans ce circuit. Le conduit 22 en serpentin de l'élément expansible fait partie de ce circuit fluidique 34.

De manière avantageuse, le module de refroidissement 30 peut être un module à refroidissement thermoélectrique. Il peut notamment s'agir d'un module à effet Peltier. Pour rappel, un module à refroidissement thermoélectrique est destiné à transformer un courant électrique en une différence de température, et l'effet Peltier est un effet thermoélectrique consistant en un phénomène physique de déplacement de chaleur en présence d'un courant électrique.

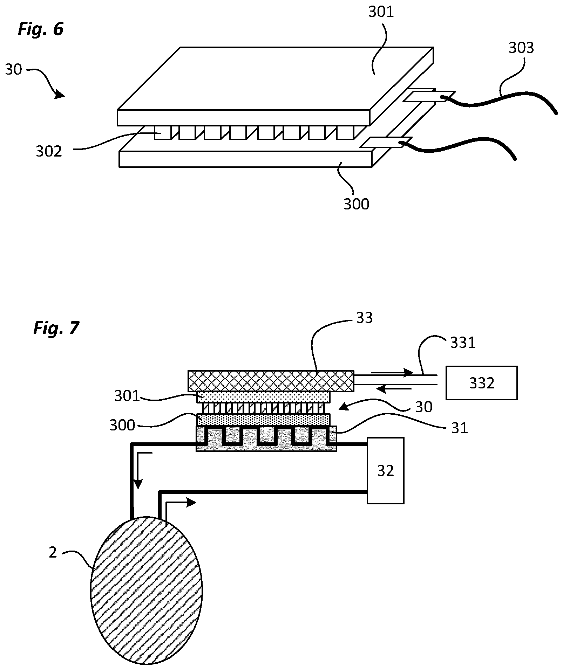

En référence à la figure 6, un module à refroidissement thermoélectrique est plus précisément une cellule fabriquée avec des composants semi-conducteurs 302 asymétriques. Ils sont connectés thermiquement en parallèle et électriquement en série entre une plaque 300 dite froide et une plaque 301 dite chaude. Ces deux plaques sont en général fabriquées en céramique. Les semi-conducteurs sont de type P et N. La plaque froide 300 est refroidie par l'absorption d'énergie due au passage des électrons d'un semi-conducteur à l'autre. La plaque chaude 301 récupère l'énergie thermique captée de la plaque froide 300. Il est donc impératif d'évacuer cette chaleur pour qu'elle ne réchauffe pas à nouveau le côté froid.

Le module à effet Peltier comporte avantageusement une densité de puissance élevée (exemple 30W/cm² pour les modules TEC Microsystem 1MA10-071-03), un bon rendement et une bonne fiabilité, afin d'envisager son intégration dans un système particulièrement compact et compatible avec une utilisation clinique long terme. Le module 30 est alimenté électriquement via des conducteurs électriques 303.

Pour refroidir le fluide, le circuit fluidique 34 comporte un échangeur thermique 31 coopérant avec la plaque froide 300 du module de refroidissement 30. Le circuit fluidique 34 est actionné par l'ensemble de pompage 32.

Pour dissiper la chaleur émise par le module à effet Peltier, au niveau de sa plaque chaude 301, le dispositif 1 comporte avantageusement un dissipateur thermique 33. En référence à la figure 7, le dissipateur 33 peut être directement monté sur la plaque chaude 301 du module 30 de refroidissement à effet Peltier. Le dissipateur 33 peut comporter un circuit de refroidissement 331 à liquide, pouvant être alimenté par une unité de pompage 332 dédiée. L'unité de pompage 332 peut être intégrée à l'unité de commande et d'alimentation électrique 4.

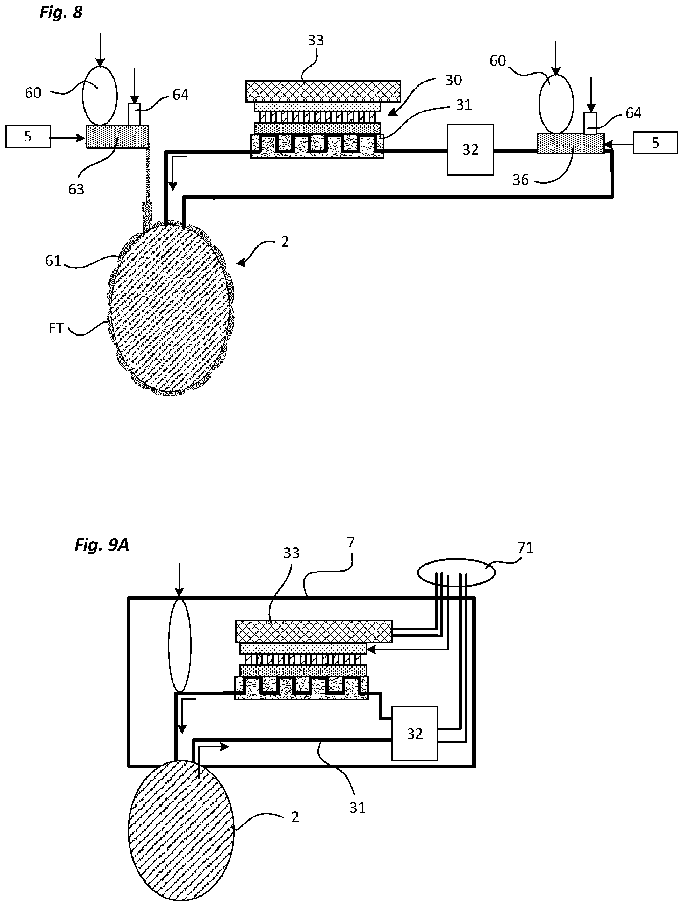

L'ensemble de pompage 32 du circuit fluidique 34 dédié au refroidissement peut comporter au moins une pompe de type mécanique, comme illustré sur la figure 9A. La pompe peut être une pompe dite centrifuge, péristaltique ou à membranes, par exemple, actionnée grâce à un circuit fluidique extérieur spécifique (commande pneumatique ou liquide). L'actionnement fluidique de la pompe permet notamment de conserver la compatibilité IRM du dispositif.

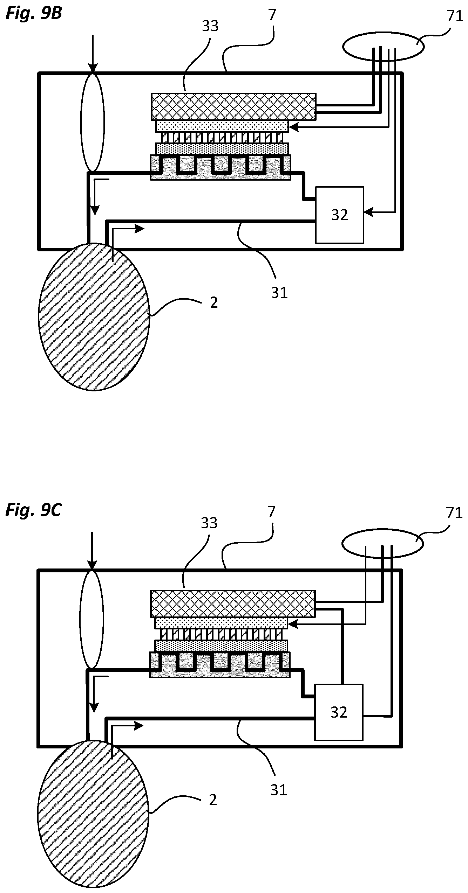

En variante représentée sur la figure 9B, la pompe peut être, par exemple, une pompe centrifuge, péristaltique, à membrane, à engrenage, excentrique, actionnée de manière électrique. Ces différents types de pompes étant bien connus, ils ne sont pas illustrés dans la présente demande.

En variante représentée sur la figure 9C, la pompe peut être, par exemple, une pompe mécanique actionnée par le circuit de refroidissement du dissipateur 33 et positionné avant celui-ci afin de réduire les pertes thermiques. Cette solution permet de réduire le nombre de composants et connexions, solution favorable à la réduction de l'encombrement et à une bonne fiabilité du système.

Avantageusement, le dispositif peut comporter un réservoir 36 venant se connecter sur le circuit fluidique 34. De manière non limitative, ce réservoir 36 peut être réalisé en accordéon et actionnable à distance grâce à l'emploi d'un actionneur 64 afin d'adapter son volume lors des phases de remplissage.

L'unité de commande et d'alimentation électrique 4 du dispositif 1 peut comporter une alimentation électrique 40 et un contrôleur 41 chargé d'envoyer des commandes, notamment :
- A l'ensemble de pompage 32 pour permettre une circulation du fluide dans le circuit fluidique 34,
- Au module de refroidissement 30 pour ajuster la température,
- A l'actionneur 64 associé au réservoir 36 pour contrôler la pression de fluide dans le circuit fluidique 34.

L'alimentation électrique permet d'alimenter les différents composants du dispositif, notamment le contrôleur 41, le module de refroidissement 30, l'ensemble de pompage 32, la pompe 332 dédiée au dissipateur thermique et éventuellement les différents moyens de surveillance détaillés ci-après. Il faut noter que l'ensemble de pompage 32 peut également être actionné de manière fluidique en employant une unité externe d'actionnement (figure 9A).

Le dispositif peut également embarquer différents moyens de surveillance 5 pour contrôler les paramètres suivants :
- Température au niveau du module de refroidissement 30 (plaques chaude et froide du module Peltier), du fluide en entrée de la pompe, et du fluide en sortie du dissipateur thermique ;
- Pression dans la boite crânienne pour éviter le risque de surpression liée au dispositif, principalement pendant le remplissage de l'élément expansible, et permettre le suivi des paramètres de pression intracrânienne tout au long du traitement ;
- Température, débit et pression du fluide dans le circuit fluidique 34 ;
- Niveau de fluide dans le réservoir 36 ;
- Mesure de pH dans le circuit fluidique 34 ;
- Mesure de turbidité, notamment dans le circuit fluidique 34 (exemple : présence de liquides biologiques dans l'élément expansible) ;

Pour garantir l'efficacité du refroidissement au niveau des berges d'exérèse sur le long terme, il faut notamment s'assurer que la surface externe de l'élément expansible reste au contact du tissu cérébral. Le dispositif de suivi du contact entre l'élément expansible et les parois de la cavité peut ainsi utiliser différents principes de fonctionnement :
- Suivi de la dissipation thermique entre l'élément expansible et le cerveau : Plus l'élément expansible 2 est en contact avec les parois de la cavité, plus la perte thermique sera importante ; une mesure différentielle de la température entre l'entrée et la sortie du conduit 22 de l'élément expansible, ainsi que de la puissance utile engagée pour obtenir un refroidissement constant peut être envisagée.
- Un contrôle par imagerie radio ou IRM ; la paroi externe de l'élément expansible 2, ou sa surface externe, peut être composée ou enduite d'un matériau radio-opaque.
- Emploi et mise en place d'électrodes de suivi d'impédance, capacitives ou inductives sur la surface externe de l'élément expansible.
- Suivi par mesure ultrasonore ou optique (DRS pour "Diffuse Reflectance Spectroscopy").

De manière avantageuse, le dispositif 1 peut également comporter des moyens de diffusion locale d'agents thérapeutiques, pour réaliser un traitement par chimiothérapie, radiothérapie ou médicaments anti-inflammatoires par exemple. L'injection de tels agents peut être mise en oeuvre en employant le circuit fluidique déjà présent, en injectant un fluide thérapeutique FT directement dans le fluide de refroidissement, ou par l'intermédiaire d'un circuit fluidique indépendant.

En cas d'ajout d'un circuit fluidique dédié, celui-ci peut comporter des capillaires rapportés sur la surface du ballonnet externe 24 de l'élément expansible 2. Comme représenté sur la figure 8, il peut également comporter un ballonnet externe 61 rapporté à l'extérieur de l'élément expansible 2 dans lequel est injecté le fluide thérapeutique FT. Il est par exemple solidaire de l'élément expansible par plusieurs points d'accroche répartis sur la surface de l'élément expansible 2 et doté d'une paroi externe poreuse pour diffuser ledit fluide thérapeutique. Ce ballonnet 61 peut être gonflé uniquement lorsque des agents thérapeutiques sont à diffuser. Un réservoir 63 spécifique contenant du fluide thérapeutique FT est par exemple connecté sur le circuit d'alimentation du ballonnet 61. Lorsque le refroidissement est commandé, ce ballonnet 61 peut être rétracté, permettant de diffuser le froid par l'élément expansible.

Dans tous les cas, les moyens vecteurs des agents thérapeutiques pourront comporter une paroi poreuse pour permettre la diffusion de ces agents.

De manière avantageuse, comme illustré sur la figure 8, les moyens 5 peuvent être chargés du suivi et de la régulation du volume de fluide thérapeutique FT qui est injecté dans le ballonnet 61. Le suivi du volume injecté peut être réalisé simplement en monitorant l'actionneur 64 d'un réservoir, par exemple réalisé en accordéon, et actionnable à distance afin d'adapter son volume lors des phases de remplissage transfert dans le ballonnet 61, ou en mesurant le débit et la pression différentielle entre l'entrée et la sortie du circuit fluidique de diffusion (capteur capacitif, par ultrasons, thermométrie différentielle...). Ces moyens 5 de suivi peuvent être reliés à l'unité de commande et d'alimentation électrique 4.

En cas de diminution du volume de fluide dans chaque circuit, le dispositif peut comporter un ou plusieurs septums 60 agencés sur le circuit correspondant et permettant un remplissage indépendant du circuit. Le remplissage peut se faire directement à travers le cuir chevelu dans le but de limiter le risque d'infection.

De manière non limitative, en référence aux figures 9A, 9B et 9C, le dispositif de refroidissement peut comporter un boîtier 7 hermétique dans lequel sont logés l'ensemble de pompage 32, le module de refroidissement 30, le dissipateur thermique 33 et éventuellement le réservoir 63 à niveau ajustable. Le conduit 22 de l'élément expansible 2 est connecté sur l'ensemble de pompage. Les différentes variantes de réalisation proposées ci-dessus sont adaptables à cet assemblage dans ce boîtier 7. De manière non limitative, l'ensemble des connexions électriques et fluidiques du dispositif peut être regroupé dans un câble 71. Pour rappel, la figure 9A et la figure 9C illustrent également l'actionnement fluidique de l'ensemble de pompage 32 et la figure 9B illustre l'actionnement électrique de l'ensemble de pompage 32.

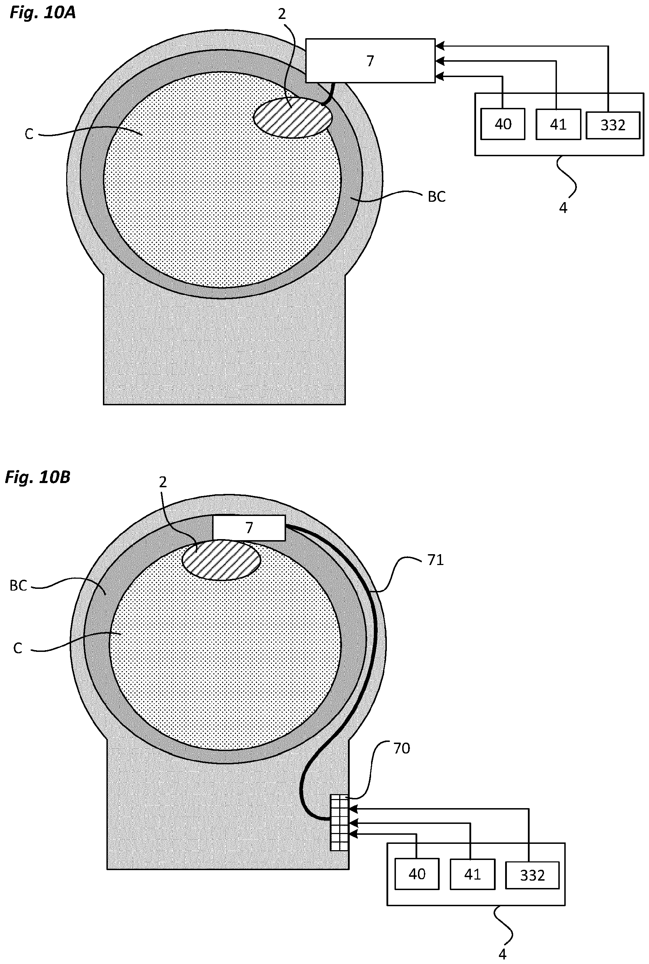

En référence aux figures 10A et 10B, il est possible d'envisager au moins deux solutions pour implanter le dispositif de l'invention :
Dans une première variante illustrée par la figure 10A, le boîtier 7 logeant le module de refroidissement 30, l'échangeur 31, le dissipateur thermique 33 ainsi que l'ensemble de pompage 32 peuvent être fixés à la boîte crânienne BC, au plus proche de la cavité d'exérèse afin de limiter les volumes morts et de réduire les pertes thermiques dans des circuits fluidiques. La plaque chaude 301 du module Peltier est alors refroidie par le dissipateur 33, par exemple réalisé sous la forme du circuit de refroidissement à liquide utilisant la pompe 332 dédiée.

Dans une deuxième variante illustrée par la figure 10B, le boîtier 7 peut être implanté directement dans la boite crânienne BC afin de faciliter le quotidien des patients et de limiter les risques d'infection. Cette variante de réalisation permet notamment d'éviter la condensation au niveau de la liaison entre le boîtier 7 et l'élément expansible 2 et donc de limiter les risques d'infection. Dans cette configuration, la zone chaude du module de refroidissement 30 peut être refroidie par une circulation fluidique gérée par le dissipateur thermique 33, alimenté par l'unité de pompage 332 externe. L'unité de pompage 332 peut être relié au dissipateur thermique 33 (situé dans la boite crânienne) grâce à un port transcutané 70 via un câble hybride (électrique-fluidique) 71. Le port transcutané 70 peut porter la connectique de commande des composants du boîtier 7 et la connectique fluidique associée au dissipateur 33 et reliée à l'unité de pompage 332 dédiée.

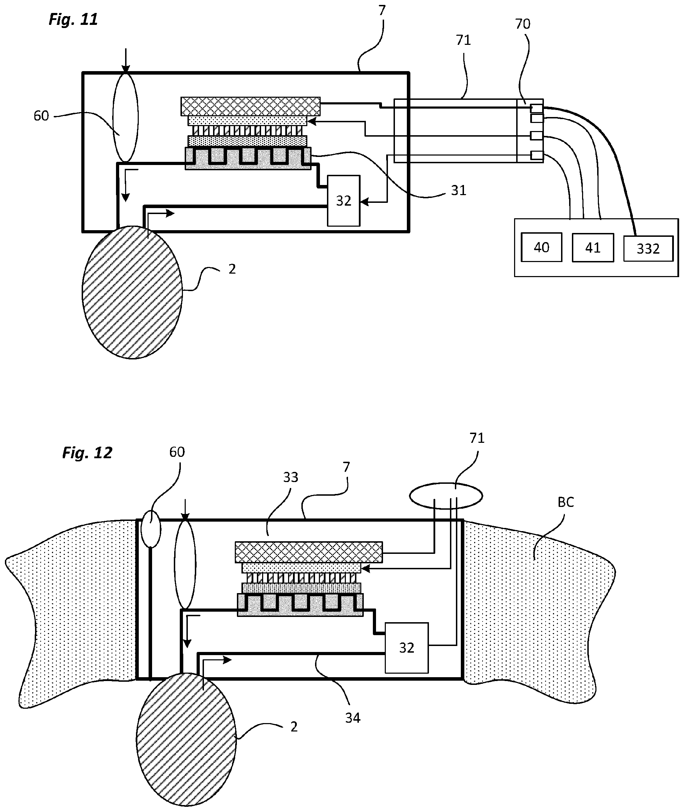

De manière générale, il est important de veiller à limiter au maximum les pertes thermiques dans ce type de système. Pour cela, le refroidissement est réalisé au voisinage de la zone à traiter avec un minimum de volumes morts. De plus, les circuits fluidiques et électriques qui peuvent être déportés le sont, en optimisant la connectique pour se limiter à un unique port transcutané. La figure 11 montre ainsi une architecture optimisée qui comporte :
- L'élément expansible 2,
- Le boîtier 7 fonctionnel,
- Un câble 71, comprenant des liaisons électriques et fluidiques,
- Un connecteur transcutané 70 sur lequel viennent se raccorder l'unité de commande et d'alimentation 4 et l'unité de pompage 332 dédiée au dissipateur 33, situées à l'extérieur du corps de l'être vivant.

Cette architecture optimisée peut être implantée comme décrit ci-dessus en liaison avec la figure 10B.

La périphérie du boîtier 7 peut être étanchéifiée grâce à du ciment dentaire afin de garantir une barrière contre les infections biologiques susceptibles de passer par le port transcutané et se propager le long du câble 71.

Dans le cas d'un implant fixé dans le crâne, comme illustré par la figure 12, on peut également envisager un système permettant de faciliter le remplissage de l'élément expansible 2, tel qu'un septum 60 dans le boitier servant d'évent intracrânien. Le système étant totalement étanche, l'évent peut permettre de gérer la pression (ponctionner en cas de surpression intracrânienne notamment) et d'éviter l'utilisation d'une chambre implantable supplémentaire.

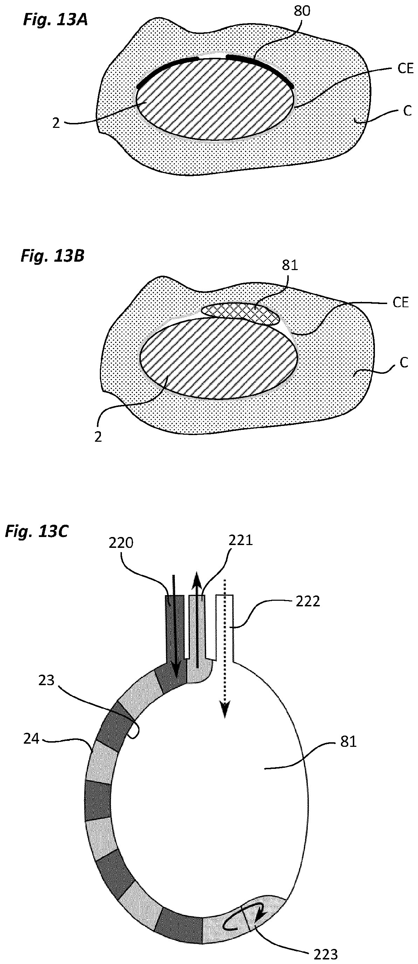

De manière avantageuse, en liaison avec les figures 13A, 13B et 13C, le dispositif 1 peut également comporter des moyens d'isolation thermique positionnés de manière localisée en une ou plusieurs zones de la surface externe de l'élément expansible 2. Ces moyens sont agencés pour isoler thermiquement l'élément expansible 2 de certaines zones de la paroi de la cavité. Ces moyens peuvent comporter une couche 80 isolante rapportée sur la surface externe de l'élément expansible (par exemple une feuille silicone ou une mousse - figure 13A) ou un ballonnet 81 gonflé à l'air venant se positionner entre l'élément expansible et la zone de la paroi de la cavité située en dehors de la zone à traiter (figure 13B). Comme illustré par la figure 13C, ce ballonnet 81 peut être avantageusement intégré au dispositif 1. Ce dispositif nécessitera l'intégration d'un filtre antibactérien à l'entrée du ballonnet ou l'usage d'un septum dédié. Il peut être gonflé via l'entrée 222 prévue et déjà décrite ci-dessus.

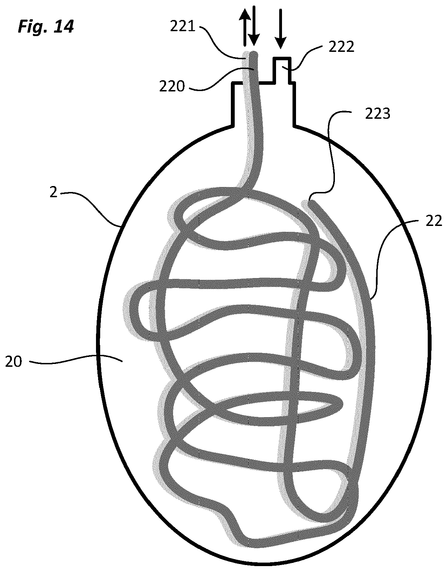

La figure 14 montre une variante de réalisation de l'élément expansible 2 employé dans le dispositif de l'invention. Dans cette variante, le conduit 22 en serpentin du circuit fluidique 34 est directement logé dans la cavité interne 20 de l'élément expansible. Dans la cavité interne 20, le conduit peut être inséré de manière aléatoire, sans forme particulière. Le conduit peut être découpé à la longueur désirée. Le conduit peut comporter deux canaux, par exemple réalisés sous la forme de deux tubes accolés ou d'un tube bi-lumen (voir description ci-dessus et figures 4D et 4E décrites ci-dessus), intégrant le circuit aller et le circuit retour. La cavité interne 20 de l'élément expansible peut être remplie par un fluide tel que du sérum physiologique, via l'entrée 222 déjà évoquée ci-dessus. Dans cette variante, le fluide de refroidissement est injecté dans le conduit 22 en serpentin et vient refroidir le fluide présent dans la cavité interne 20 de l'élément expansible 2, autour du conduit. Il permet ainsi d'obtenir un refroidissement du fluide présent dans l'élément expansible globalement homogène et uniforme. Cette variante de réalisation permet de simplifier le système en acceptant cependant une gestion de l'uniformité en température moindre. Les autres particularités du dispositif décrites ci-dessus sont applicables de manière identique à cette variante de réalisation de l'élément expansible.

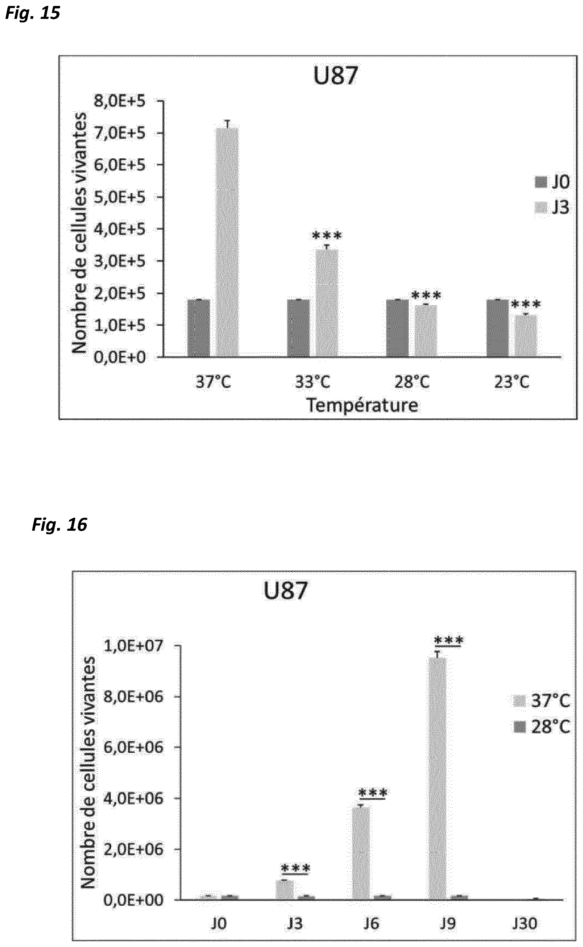

Le refroidissement d'une cavité d'exérèse CE dans le but de prévenir la récidive tumorale a été envisagé suite à des travaux *in vitro* démontrant les effets inhibiteurs de l'hypothermie sur la croissance tumorale. En liaison avec les figures 15 à 18, les résultats présentés proviennent de différentes expériences réalisées sur des lignées cellulaires provenant de glioblastome humain (publication : C. Fulbert, C. Gaude, E. Sulpice, S. Chabardès, et D. Ratel, « Moderate hypothermia inhibits both prolifération and migration of human glioblastoma cells », Journal of Neuro-Oncology, août 2019, doi: 10.1007/s11060-019-03263-3*).* Les résultats présentés ici correspondent à une seule lignée (U87), mais des résultats similaires ont été obtenus pour trois autres lignées indépendantes issues de glioblastome humain (A172, U251, T98G). Chaque diagramme annexé correspond à la moyenne d'au moins trois expériences réalisées indépendamment les unes des autres et les résultats sont présentés de la manière suivante : moyenne ± erreur type de la moyenne (SEM). Le seuil de significativité statistique est fixé à une valeur-p inférieure à 0,05, et le nombre d'étoiles correspond au degré de significativité statistique : **p < 0,01 ; ***p < 0,001.

Il a été déjà démontré que l'hypothermie avait des effets inhibiteurs sur la prolifération et la migration des cellules de glioblastome. D'abord, l'inhibition de la prolifération est température dépendante, avec une inhibition partielle à 33°C et totale en dessous de 28°C (figure 15). Ensuite, le blocage de la prolifération cellulaire à 28°C est durable dans le temps, jusqu'à au moins 30 jours (figure 16).

Ainsi, l'hypothermie est capable de stopper définitivement la prolifération de cellules de glioblastome lorsqu'elle est appliquée de manière continue.

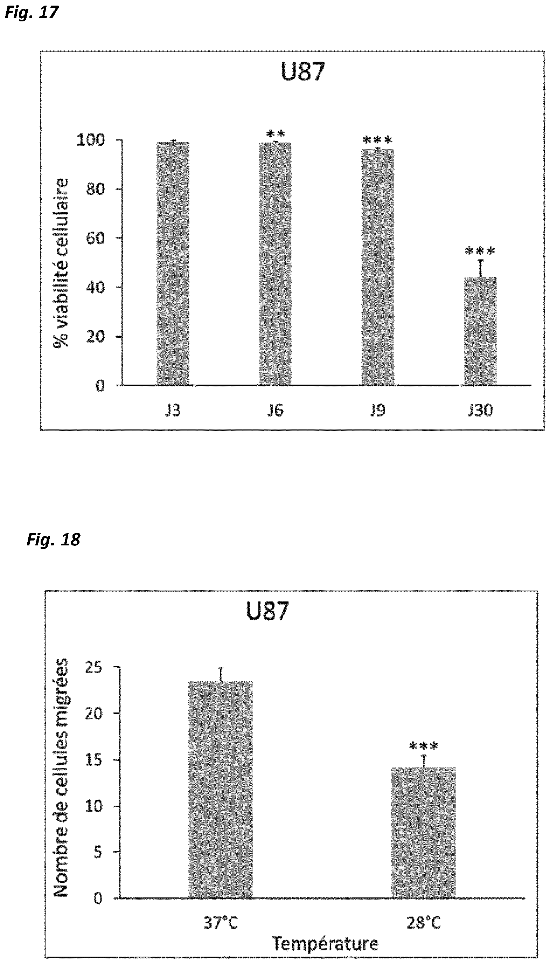

De plus, il a été démontré qu'une hypothermie de 28°C induisait la mort de cellules de glioblastome après 30 jours de culture (figure 17), et qu'elle diminuait leur migration de manière significative (figure 18).

Ainsi, ces résultats démontrent un fort potentiel thérapeutique de l'hypothermie dans le blocage du développement tumoral, et notamment de la récidive.

L'hypothermie pourrait également permettre de potentialiser les effets de la radiothérapie et de la chimiothérapie. En effet, une étude *in vivo* menée sur des souris a montré que la combinaison de l'hypothermie avec un agent chimiothérapeutique permettait d'améliorer la survie des animaux et de diminuer les effets cytotoxiques du médicament (publication : R. Hultborn, L. Lundgren-Eriksson, S. Ottosson-Lönn, W. Ryd, et L. Weiss, « Chlorpromazine-induced hypothermia in tumour-bearing mice, acute cytotoxic drug lethality and long-term survival », Acta Oncol, vol. 29, n° 7, p. 941-944, 1990, doi: 10.3109/02841869009096394*).* D'autre part, l'hypothermie a également été envisagée comme traitement adjuvant de la radiothérapie dans plusieurs études expérimentales puisqu'elle permet d'augmenter l'oxygénation des tumeurs, et de ce fait leur radiosensibilité (voir publications listées ci-dessous).

Publications :
- A. H. Nias, P. M. Perry, et A. R. Photiou, « Modulating the oxygen tension in tumours by hypothermia and hyperbaric oxygen », J R Soc Med, vol. 81, no 11, p. 633-636, nov. 1988*.*
- M.-A. Neveu, N. Joudiou, G. D. Preter, J.-P. Dehoux, B. F. Jordan, et B. Gallez, « 17O MRS assesses the effect of mild hypothermia on oxygen consomption rate in tumors », NMR in Biomedicine, vol. 30, no 8, p. e3726, 2017, doi: 10.1002/nbm.3726*.*
- D. K. Kelleher, C. Nauth, O. Thews, W. Krueger, et P. Vaupel, « Localized hypothermia: impact on oxygénation, microregional perfusion, metabolic and bioenergetic status of subcutaneous rat tumours », Br. J. Cancer, vol. 78, no 1, p. 56-61, juill. 1998, doi: 10.1038/bjc. 1998.442*.*

Le dispositif présente ainsi de nombreux avantages, parmi lesquels :
- Son élément expansible est facilement implantable dans une cavité d'exérèse ;
- Il permet de garantir l'obtention d'une température homogène au niveau des parois de la cavité d'exérèse ;
- L'élément expansible peut être conformable à la forme de la cavité, par exemple grâce à l'emploi de matériaux souples et fins au niveau de l'élément expansible et/ou par thermoformage ;
- Il peut disposer de divers moyens de suivi et de régulation, permettant d'optimiser son fonctionnement ;
- Il dispose de moyens pour remplir le circuit fluidique (septum) en garantissant la barrière stérile;
- Il permet d'optimiser la puissance de refroidissement, notamment en réduisant les pertes thermiques ;
- Il est portable et compatible avec le quotidien d'un patient sur le long terme (> 30 jours) ;
- Il est compatible avec une prise en charge thérapeutique du patient, notamment grâce aux moyens permettant l'injection d'agents thérapeutiques ;
- Il est compatible avec un suivi médical du patient, via IRM en particulier ;
- Il permet de minimiser les risques infectieux pour le patient ;

## Revendications

1. Dispositif (1) de refroidissement localisé comprenant un élément expansible destiné à occuper le volume d'une cavité d'exérèse suite à l'ablation chirurgicale d'une tumeur réalisée chez un être vivant, ledit élément expansible (2) présentant une paroi externe destinée à venir en contact au moins partiellement avec les parois de la cavité, le dispositif étant **caractérisé en ce qu'**il comporte :
- Une unité de refroidissement (3) comprenant un circuit fluidique (34) dans lequel peut circuler un fluide de refroidissement, un module de refroidissement (30) coopérant avec ledit circuit fluidique et au moins un ensemble de pompage (32) connecté sur ledit circuit fluidique pour faire circuler le fluide dans ledit circuit fluidique (34),
- Une unité de commande et d'alimentation électrique (4) pour commander et alimenter ladite unité de refroidissement (3),
- Ledit élément expansible (2) comportant une cavité interne (20) délimitée par une paroi (21),
- Un conduit (22) en serpentin, appartenant au circuit fluidique et rapporté sur ladite paroi (21), formant ladite paroi (21) de l'élément expansible, ou logé dans la cavité interne (20) de l'élément expansible.

2. Dispositif selon la revendication 1, **caractérisé en ce que** ladite paroi (21) de la cavité interne de l'élément expansible (2) présente une structure alvéolée réalisée par le conduit (22) en serpentin.

3. Dispositif selon la revendication 2, **caractérisé en ce que** le conduit (22) en serpentin est réalisé sous la forme de deux spirales imbriquées, de manière à former ladite structure alvéolée.

4. Dispositif selon la revendication 2 ou 3, **caractérisé en ce que** le conduit (22) en serpentin est réalisé sous la forme d'un tube bi-lumen.

5. Dispositif selon l'une des revendications 2 à 4, **caractérisé en ce que** l'élément expansible comporte un ballonnet interne (23) présentant une surface externe sur laquelle est rapporté ledit conduit (22) en serpentin et un ballonnet externe (24) venant recouvrir ledit conduit (22) en serpentin.

6. Dispositif selon l'une des revendications 2 à 5, **caractérisé en ce que** le conduit (22) en serpentin comporte une entrée (220) et une sortie (221) et **en ce que** sa sortie (221) débouche dans ladite cavité interne (20) de l'élément expansible (2).

7. Dispositif selon l'une des revendications 1 à 6, **caractérisé en ce qu'**il comporte également un circuit indépendant de diffusion d'un fluide thérapeutique connecté sur ledit ensemble de pompage (32) et agencé sur ou autour de la paroi externe dudit élément expansible (2).

8. Dispositif selon la revendication 7, **caractérisé en ce que** ledit circuit de diffusion de fluide thérapeutique comporte un conduit rapporté sur la paroi externe dudit élément expansible.

9. Dispositif selon la revendication 7, **caractérisé en ce que** ledit circuit de diffusion de fluide thérapeutique comporte un ballonnet (6) externe solidaire dudit élément expansible (2).

10. Dispositif selon l'une des revendications 1 à 9, **caractérisé en ce que** l'élément expansible (2) est thermoformé pour présenter une forme externe de dimensions identiques à celles de la cavité d'exérèse.

11. Dispositif selon l'une des revendications 1 à 10, **caractérisé en ce que** le module de refroidissement (30) comporte un module à effet Peltier.

12. Dispositif selon l'une des revendications 1 à 11, **caractérisé en ce qu'**il comporte des moyens (5) de suivi de la température du fluide et du module de refroidissement (30), de débit et de pression de fluide de refroidissement dans le circuit fluidique (34), et de volume et de la turbidité du fluide de refroidissement.

13. Dispositif selon l'une des revendications 1 à 12, **caractérisé en ce que** l'ensemble de pompage (32) est actionné de manière fluidique.

14. Dispositif selon l'une des revendications 1 à 13, **caractérisé en ce qu'**il comporte un boîtier (7) hermétique logeant le module de refroidissement (30) et l'ensemble de pompage (32).

15. Dispositif selon la revendication 14, **caractérisé en ce que** l'élément expansible (2) est fixé audit boîtier (7), un câble (71) reliant ledit boîtier à l'unité de commande et d'alimentation (4), via un connecteur transcutané (70).

16. Dispositif selon l'une des revendications 1 à 15, **caractérisé en ce qu'**il comporte un réservoir (36) destiné à recevoir un fluide et relié au circuit fluidique (34), et un actionneur pilotable par l'unité de commande et d'alimentation (4) pour régler le niveau de fluide dans le réservoir.

17. Dispositif selon l'une des revendications 1 à 16, **caractérisé en ce qu'**il comporte des moyens d'isolation thermique (80, 81) positionnés contre au moins une partie de la paroi externe de l'élément expansible (2).

## Patentansprüche

1. Vorrichtung (1) zur lokalisierten Kühlung, welche ein ausdehnbares Element umfasst, das dazu bestimmt ist, das Volumen eines Resektionshohlraums im Anschluss an die an einem Lebewesen durchgeführte chirurgische Entfernung eines Tumors einzunehmen, wobei das ausdehnbare Element (2) eine Außenwand aufweist, die dazu bestimmt ist, wenigstens teilweise mit den Wänden des Hohlraums in Kontakt zu kommen, wobei die Vorrichtung **dadurch gekennzeichnet ist, dass** sie aufweist:
- eine Kühleinheit (3), die einen Fluidkreislauf (34), in dem ein Kühlfluid zirkulieren kann, ein Kühlmodul (30), das mit dem Fluidkreislauf zusammenwirkt, und mindestens eine Pumpenanordnung (32), die an den Fluidkreislauf angeschlossen ist, um das Fluid im Fluidkreislauf (34) zirkulieren zu lassen, umfasst,
- eine Steuer- und Stromversorgungseinheit (4) zur Steuerung und Stromversorgung der Kühleinheit (3),
- wobei das ausdehnbare Element (2) einen inneren Hohlraum (20) aufweist, der von einer Wand (21) begrenzt wird,
- eine schlangenförmige Leitung (22), die zum Fluidkreislauf gehört und an die Wand (21) angesetzt ist, wobei sie die Wand (21) des ausdehnbaren Elements bildet, oder im inneren Hohlraum (20) des ausdehnbaren Elements aufgenommen ist.

2. Vorrichtung nach Anspruch 1, **dadurch gekennzeichnet, dass** die Wand (21) des inneren Hohlraums des ausdehnbaren Elements (2) eine Wabenstruktur aufweist, die durch die schlangenförmige Leitung (22) hergestellt wird.

3. Vorrichtung nach Anspruch 2, **dadurch gekennzeichnet, dass** die schlangenförmige Leitung (22) in Form von zwei ineinander verschachtelten Spiralen ausgeführt ist, um so die Wabenstruktur zu bilden.

4. Vorrichtung nach Anspruch 2 oder 3, **dadurch gekennzeichnet, dass** die schlangenförmige Leitung (22) in Form eines Doppellumentubus ausgeführt ist.

5. Vorrichtung nach einem der Ansprüche 2 bis 4, **dadurch gekennzeichnet, dass** das ausdehnbare Element einen inneren Ballon (23), der eine Außenfläche aufweist, auf der die schlangenförmige Leitung (22) angesetzt ist, und einen äußeren Ballon (24), der die schlangenförmige Leitung (22) bedeckt, aufweist.

6. Vorrichtung nach einem der Ansprüche 2 bis 5, **dadurch gekennzeichnet, dass** die schlangenförmige Leitung (22) einen Eingang (220) und einen Ausgang (221) aufweist, und dadurch, dass ihr Ausgang (221) in den inneren Hohlraum (20) des ausdehnbaren Elements (2) mündet.

7. Vorrichtung nach einem der Ansprüche 1 bis 6, **dadurch gekennzeichnet, dass** sie außerdem einen unabhängigen Kreislauf zur Diffusion eines therapeutischen Fluids aufweist, der an die Pumpenanordnung (32) angeschlossen ist und an der Außenwand des ausdehnbaren Elements (2) oder um diese herum angeordnet ist.

8. Vorrichtung nach Anspruch 7, **dadurch gekennzeichnet, dass** der Kreislauf zur Diffusion eines therapeutischen Fluids eine Leitung aufweist, die an die Außenwand des ausdehnbaren Elements angesetzt ist.

9. Vorrichtung nach Anspruch 7, **dadurch gekennzeichnet, dass** der Kreislauf zur Diffusion eines therapeutischen Fluids einen äußeren Ballon (6) aufweist, der mit dem ausdehnbaren Element (2) fest verbunden ist.

10. Vorrichtung nach einem der Ansprüche 1 bis 9, **dadurch gekennzeichnet, dass** das ausdehnbare Element (2) warmgeformt ist, so dass es eine äußere Form mit Abmessungen aufweist, die mit denjenigen des Resektionshohlraums identisch sind.

11. Vorrichtung nach einem der Ansprüche 1 bis 10, **dadurch gekennzeichnet, dass** das Kühlmodul (30) ein Peltier-Effekt-Modul aufweist.

12. Vorrichtung nach einem der Ansprüche 1 bis 11, **dadurch gekennzeichnet, dass** sie Mittel (5) zur Verfolgung der Temperatur des Fluids und des Kühlmoduls (30), der Durchflussmenge und des Drucks des Kühlfluids im Fluidkreislauf (34) und des Volumens und der Trübung des Kühlfluids aufweist.

13. Vorrichtung nach einem der Ansprüche 1 bis 12, **dadurch gekennzeichnet, dass** die Pumpenanordnung (32) fluidisch betätigt wird.

14. Vorrichtung nach einem der Ansprüche 1 bis 13, **dadurch gekennzeichnet, dass** sie ein hermetisches Gehäuse (7) aufweist, welches das Kühlmodul (30) und die Pumpenanordnung (32) aufnimmt.

15. Vorrichtung nach Anspruch 14, **dadurch gekennzeichnet, dass** das ausdehnbare Element (2) am Gehäuse (7) befestigt ist, wobei ein Kabel (71) das Gehäuse über einen transkutanen Verbinder (70) mit der Steuer- und Stromversorgungseinheit (4) verbindet.

16. Vorrichtung nach einem der Ansprüche 1 bis 15, **dadurch gekennzeichnet, dass** sie einen Behälter (36), der dazu bestimmt ist, ein Fluid aufzunehmen, und mit dem Fluidkreislauf (34) verbunden ist, und einen Aktuator, der durch die Steuer- und Stromversorgungseinheit (4) steuerbar ist, um den Fluidpegel im Behälter zu regeln, aufweist.

17. Vorrichtung nach einem der Ansprüche 1 bis 16, **dadurch gekennzeichnet, dass** sie Wärmeisolationsmittel (80, 81) aufweist, die an wenigstens einem Teil der Außenwand des ausdehnbaren Elements (2) anliegend positioniert sind.

## Claims

1. Localized cooling device (1) comprising an expandable element intended to occupy the volume of an excision cavity following the surgical ablation of a tumour performed on a living being, said expandable element (2) having an outer wall intended to come into contact at least partially with the walls of the cavity, the device being **characterized in that** it comprises:
- a cooling unit (3) comprising a fluidic circuit (34) in which a coolant can circulate, a cooling module (30) cooperating with said fluidic circuit and at least one pumping assembly (32) connected to said fluidic circuit to make the fluid circulate in said fluidic circuit (34),
- a control and electrical power supply unit (4) for controlling and powering said cooling unit (3),
- said expandable element (2) comprising an internal cavity (20) delimited by a wall (21),
- a coiled duct (22), belonging to the fluidic circuit and added onto said wall (21), forming said wall (21) of the expandable element, or housed in the internal cavity (20) of the expandable element.

2. Device according to Claim 1, **characterized in that** said wall (21) of the internal cavity of the expandable element (2) has a cellular structure produced by the coiled duct (22).

3. Device according to Claim 2, **characterized in that** the coiled duct (22) is produced in the form of two nested spirals, so as to form said cellular structure.

4. Device according to Claim 2 or 3, **characterized in that** the coiled duct (22) is produced in the form of a bi-lumen tube.

5. Device according to one of Claims 2 to 4, **characterized in that** the expandable element comprises an internal balloon (23) having an outer surface onto which is added said coiled duct (22) and an outer balloon (24) covering said coiled duct (22).

6. Device according to one of Claims 2 to 5, **characterized in that** the coiled duct (22) comprises an input (220) and an output (221) and **in that** its output (221) merges into said internal cavity (20) of the expandable element (2).

7. Device according to one of Claims 1 to 6, **characterized in that** it also comprises an independent circuit for diffusion of a therapeutic fluid connected to said pumping (2).

8. Device according to Claim 7, **characterized in that** said therapeutic fluid diffusion circuit comprises a duct added onto the outer wall of said expandable element.

9. Device according to Claim 7, **characterized in that** said therapeutic fluid diffusion circuit comprises an outer balloon (6) secured to said expandable element (2).

10. Device according to one of Claims 1 to 9, **characterized in that** the expandable element (2) is thermoformed to have an outer form of dimensions identical to those of the excision cavity.

11. Device according to one of Claims 1 to 10, **characterized in that** the cooling module (30) comprises a Peltier-effect module.

12. Device according to one of Claims 1 to 11, **characterized in that** it comprises means (5) for tracking the temperature of the fluid and of the cooling module (30), the flow rate and pressure of coolant in the fluidic circuit (34), and the volume and turbidity of the coolant.

13. Device according to one of Claims 1 to 12, **characterized in that** the pumping assembly (32) is actuated fluidically.

14. Device according to one of Claims 1 to 13, **characterized in that** it comprises a hermetic housing (7) housing the cooling module (30) and the pumping assembly (32).

15. Device according to Claim 14, **characterized in that** the expandable element (2) is fixed to said housing (7), a cable (71) linking said housing to the control and power supply unit (4), via a transcutaneous connector (70).

16. Device according to one of Claims 1 to 15, **characterized in that** it comprises a tank (36) intended to receive a fluid and linked to the fluidic circuit (34), and an actuator that can be driven by the control and power supply unit (4) to set the fluid level in the tank.

17. Device according to one of Claims 1 to 16, **characterized in that** it comprises thermal insulation means (80, 81) positioned against at least a part of the outer wall of the expandable element (2).
